# EUROPEAN PATENT SPECIFICATION

(11) **EP 1 572 696 B1**
(45) Date of publication and mention of the grant of the patent: **14.06.2006**
(21) Application number: 02788386.7
(22) Date of filing: 16.12.2002
(51) Int. Cl.: C07D 491/22, C07D 491/14, C07D 311/00, C07D 221/00, C07D 209/00

(54) **PROCESS FOR THE DIRECT PREPARATION OF 5-ALKOXY AND 5-ACYLOXY ANALOGUES OF CAMPTHOTHECINS OR MAPPICENE KETONES**
VERFAHREN ZUR DIREKTEN HERSTELLUNG VON 5-ALKOXY UND 5-ACYLOXY ANALOGEN VON CAMPTHOTHECIN ODER MAPPICENE KETON
PROCEDE DE PREPARATION DIRECTE D'ANALOGUES DE 5-ALCOXY ET DE 5-ACYLOXY DE CAMPTOTHECINES OU DE MAPPICINES CETONES

(43) Date of publication of application: 14.09.2005
(73) Proprietor: COUNCIL OF SCIENTIFIC AND INDUSTRIAL RESEARCH, New Delhi 110 001 (IN)
(72) Inventor: DAS, Biswanath, Hyderabad 500 007 (Andhra Pradesh) (IN); MADHUSUDAN, Purushotham, Hyderabad 500 007 (Andhra Pradesh) (IN); KALAVAGUNTA, Venkata Naga Satya Srinivas, Hyderabad 500 007 (Andhra Pradesh) (IN)
(74) Representative: Geary, Stephen
(86) International application number: PCT/IB2002/005510
(87) International publication number: WO 2004/055020

(56) References cited:
- EP-A- 1 138 682
- US-A- 3 894 029
- US-A- 5 734 056
- SUBRAHMANYAM D D ET AL: "Novel C-ring analogues of 20(S)-camptothecin. Part 3: synthesis and their in vitro cytotoxicity of A-, B- and C-ring analogues" BIOORGANIC & MEDICINAL CHEMISTRY LETTERS, OXFORD, GB, vol. 10, no. 4, February 2000 (2000-02), pages 369-371, XP004189935 ISSN: 0960-894X cited in the application

## Description

### Field of the invention

The present invention relates to a method for direct preparation of 5-alkoxy and 5-acyloxy analogues of camptothecins or mappicine ketones.

### Background of the invention

Camptothecin **(I)** is a naturally occurring pyrrolo (3,4-b)-quinoline alkaloid having remarkable antitumour and antileukemic activities (Wall et. al., *J.Am.Chem.Soc.* **88**, 3888, 1966). It acts as an inhibitor of DNA topoisomerase I (Hsiang et al., *J.Biol.Chem.,* **260**, 14873, 1985). Two natural analogues of the compound, 9-methoxy camptothecin **(II)** (Govindachari and Viswanathan, *Phytochemistry,* **11**, 3529, 1972; Wu et al., *Phytochemistry,* **39**, 383, 1995) and 20-O-acetyl camptothecin **(III)** (Wu et al., *Phytochemistry,* **39**, 383, 1995; Das et al., *Indian J. Chem,* **36B**, 207, 1997) also possess significant cytotoxic property. The decarboxylated E-ring analogues of camptothecin **(I)** and 9-methoxycamptothecin (**II**), known as mappicine ketone (nothapodytine B) (**IV**) and 9-methoxymappicine ketone ( nothapodytine A) (**V**) respectively are also naturally occurring alkaloids (Wu et al., *Phytochemistry,* **42,** 907, 1996; Pendrak et al., *J.Org.Chem,* **59,** 2623, 1994). Mappicine ketone (**IV**) has recently been identified as an antiviral lead (Pendrak et al., *J.Org.Chem.,* **59,** 2623, 1994; *J.Org.Chem.* **60,** 2912, 1995).

As a drug camptothecin (**I**) itself has several problems including its high toxicity and low water solubility. So several analogues of the compound have been prepared to evaluate their clinical efficacies. Recently different 5-substituted alkoaycamptothecins have been found to exhibit potent anticancer activity (Subrahmanyam et.al., *Bioorg. Med,Chem.Lett.,* **10**, 369, 2000). The methods of preparation of 5-alkoxy camptothecins are limited. They were prepared earlier from 5-hydroxycamptothecin which was obtained by treatment of camptothecin with I₂ and K₂CO₃ in DMF. 5-Hydroxycamptothecin was subsequently converted into 5-alkoxycamptothecins by reacting with alcohols in the presence of BF₃.OEt₂ ( Swada et al., *Chem.Pharm Bull.,* **39**,2574,1991). This is a two-step process and so the overall yields of 5-alkoxycamptothecins will be diminished. The experimental procedures are also tidious. In the other approach camptothecin was heated with FeCh-H₂SO₄ in EtOH for 20 hr to obtain a mixture of 5-ethoxy and 5-hydroxycamptothecins. The mixture was again heated with aqueous HCl for 20 hr to obtain 5-hydroxycamptothecin exclusively. This 5-hydroxycamptothecin was then treated with different alcohols in the presence of p-toluenesulfonic acid to prepare 5-alkoxycamptothecins (Subrahmanyam et al., *Biorg.Med Chem Lett.,* **10**, 369, 2000). This method involves several steps and the time required is high.

### Object of the invention

The main object of the invention is to provide a convenient and facile process for the direct preparation of 5-alkoxy and 5-acyloxy analogues of campthothecins and mappicene ketones.

It is another object of the invention to provide a process for direct preparation of 5-alkoxy and 5-acyloxy analogues of campthothecins and mappicene ketones in high yield.

It is yet another object of the invention to provide a process for direct preparation of 5-alkoxy and 5-acyloxy analogues of campthothecins and mappicene ketones which is simple and non-hazardous.

### Summary of the invention

Accordingly the present invention provides a process for the direct preparation of 5-alkoxy and 5-acyloxy analogues of campthothecins or mappicene ketones comprising reacting campthothecin or mappicene ketone dissolved in an organic solvent or an acid with ceric ammonium nitrate in an organic solvent at a temperature in the range of 20 to 30°C for a period in the range of 2 to 10 hours, concentrating the reaction mixture and adding water to the concentrate followed by extraction with ethyl acetate to obtain the desired product.

In one embodiment of the invention, the campthothecin is selected from the group consisting of campthothecin (I), 9-methyl campthothecin (II) and 20-O-acetyl campthothecin (III).

In another embodiment of the present invention, the mappicene ketone used comprises 9-methoxymappicene ketone.

In yet another embodiment of the invention, the organic solvent used for the campthothecin solution is selected from the group consisting of dichloromethane, chloroform, tetrahydrofuran and any mixture thereof

In a further embodiment of the invention, the organic acid used for the campthothecin solution is selected from acetic acid, propanoic acid and benzoic acid.

In another embodiment of the invention, the organic solvent used for the ceric ammonium nitrate solution is selected from the group consisting of methanol, propanol, isopropanol and ethanol.

In yet another embodiment of the invention, the analogues obtained comprise the group of 5-methoxy campthothecin, 5-isopropoxy campthothecin, 5-ethoxy-9-methoxy campthothecin, 5-methoxy mappicene ketone, 5-ethoxy-9-methoxy mappicene ketone, 5-acetoxy campthothecin, 5-acetoxy-9-methoxy campthothecin, 5-propionoxy-20-O-acetylcampthothecin, 5-benzoyloxymappicene ketone and 5-acetoxy-9- methoxymappicene ketone.

### Detailed description of the invention

The present investigation has led to the development of a convenient and facile method for direct preparation of 5-alkoxy and 5-acyloxy analogues of campthothecins and mappicine ketones. Camptothecin (**I**), 9-methoxycamptothecin **(II)** and 20-O-acetylcamptothecin **(III)** as well as mappicine ketone (**IV**) and 9-methoxymappicine ketone **(V)** were isolated from the stems of Indian *Nothapodytes foetida.* Camptothecins and mappicine ketones on treatment with alcohols in the presence of ceric ammonium nitrate (CAN) at room temperature for 2 hr afforded 5-alkoxy compounds in very high yields (72-86%). Both primary and secondary alcohols were used. 5-Alkoxycamptothecins were found (from their ^{I}H NMR spectra) to be mixtures (1:1) of both the diastereoisomers with 20-(S), 5-(S) and 20-(S), 5-(R) configurations.

The mixtures were not further separated since anticancer activity was reported earlier on the mixtures (Subrahmanyam et.al., *Bioorg.Med Chem.Lett,* **10,** 369, 2000). However, for mappicine ketones each 5-alkoxy analogue was obtained as a racemic compound. During the present study several 5-acyloxy analogues of camptothecins and mappicine ketones have also been prepared.

Previously some 5-acyloxycamptothecins were prepared in two steps from camptothecins via their 5-hydroxy derivatives (Swada et al., *Chem.Pharm Bull.,* **39,** 2574, 1991). It is observed that when camptothecins were treated with acids (both aliphatic and aromatic) in the presence of CAN at room temperature for 8 hr 5-acyloxycamptothecins were obtained (as 1:1 diastereoisomeric mixtures). Hydroxy group at C-20 remained unaffected. Mappicine ketones also underwent similar conversion within 6 hr into 5-acyloxy analogues when treated with acids in the presence of CAN. The yields of the 5-acyloxycamptothecins and mappicine ketones were very high (71-82%).

The present procedure for the preparation of useful 5-substituted alkoxy and acyloxy derivatives of camptothecins and mappicine ketones is very simple and efficient. The conversion is completed within a short period of time. The reaction proceeds at room temperature and not involves any hazardous and costly reagent. The yields of the products are very high. Thus the process of the invention is useful and practical for the preparation of 5-alkoxy and 5-acyloxy analogues of camptothecins and mappicine ketones. All the analogues of mappicine ketones are new compounds.

The time taken for obtaining the products is about 2 hours for 5-alkoxy compounds and 6-8 hours for 5-acyloxy compounds. Another significant advantage of the invention is that the yields of the products are high (72-86% for 5-alkoxy compounds and 71-82% for 5-acyloxy compounds). In addition, the solvents used for extraction can be recovered rendering the process more economic. A process as claimed in Claims 1-6 wherein a library of 5-alkoxy and 5-acyloxycamptothecins and mappicine ketones can easily be generated to discover promising anticancer and anti-viral agents.

The prepared 5-alkoxy and 5-acyloxy analogues of camptothecins and mappicine ketones are useful for preparation of different other analogues which in turn are useful for discovery of novel and potent anticancer and antiviral drugs.

The reaction mechanism is given below:

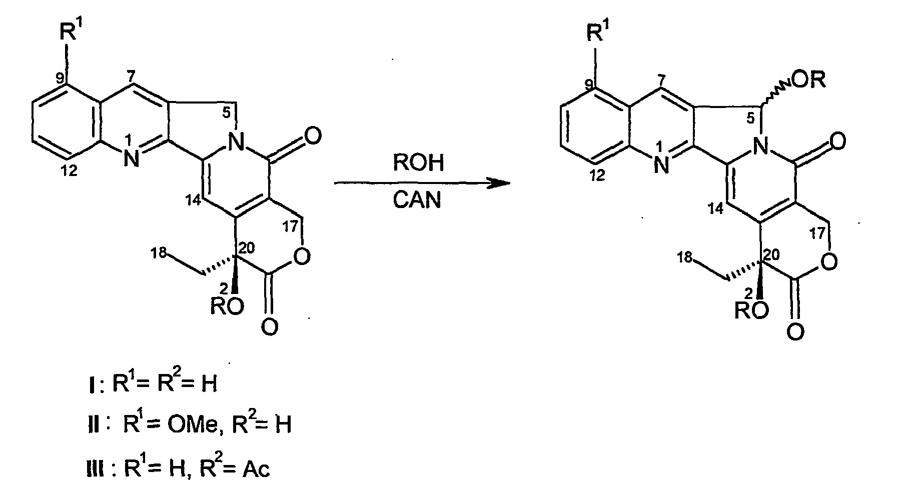

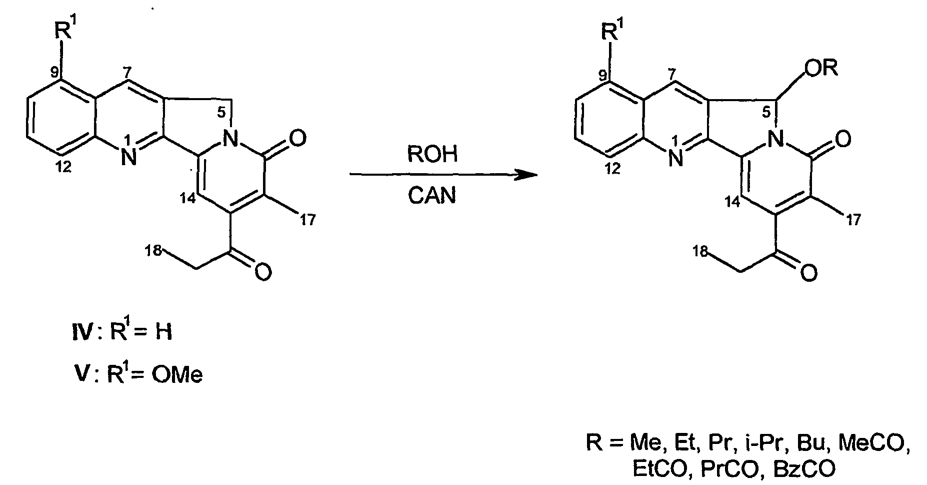

The present invention is now described with reference to the following examples which are illustrative and should not be construed as limiting the scope of the invention in any manner.

### Example: 1

To a well stirred solution of CAN (100 mg) in MeOH (10 ml) camptothecin (**I**) (100 mg) in THF-CH₂Cl₂ (1:1) (20 ml) was added. The mixture was stirred at room temperature for 2 hr. After concentration, water (20 ml) was added. The mixture was extracted with EtOAc (3x10 ml) and concentrated. Impurities were removed by column chromatography over silica gel to obtain 5-methoxycamptothecin (88 mg).

### Example: 2

Camptothecin (**I**) (100 mg) in a mixture of THF-CH₂Cl₂ (1:1) (20 ml) was added to a solution of CAN (100 mg) in isopropanol (10 ml) and the mixture was stirred at room temperature for 2 hr. The mixture was concentrated and water (20 ml) was added. This was extracted with EtOAc (3x10 ml) and concentrated. The residue was subjected to column chromatography over silica gel for removal of the impurities to afford 5-isopropoxycamptothecin (84 mg).

### Example: 3

9-Methoxycamptothecin **(II)** (100 mg) in CH₂Cl₂ (20 ml) was added to a well stirred solution of CAN (100 mg) in EtOH (10 ml). The stirring was continued at room temperature for 2 hr. The mixture was concentrated and water (20 ml) was added. This was extracted with EtOAc (3X10 ml). The concentrated extract on column chromatography over silica gel produced 5-ethoxy-9-methoxycamptothecin (81 mg).

### Example: 4

A solution of CAN (100 mg) in MeOH (10 ml) was stirred at room temperature. A solution of 20-O-acetylcamptothecin **(III)** (100 mg) in CHCl₃ ( 20 ml) was added. The mixture was stirred for 2 hr. This was concentrated and water ( 20 ml) was added. The mixture was extracted with EtOAc (3x10 ml), concentrated and subjected to column chromatography over silica gel to yield 5-methoxy-20-O-acetyl camptothecin (82 mg).

### Example: 5

To a well-stirred solution of CAN (100 mg) in MeOH (10 ml) mappicine ketone (100 mg) in CHCl₃ (20 ml) was added. The mixture was stirred at room temperature for 2 hr. The mixture was concentrated and water (20 ml) was added to this. This was extracted with EtOAc (3x10 ml). The concentrated extract was purified by column chromatography over silica gel to yield 5-methoxy mappicine ketone (95 mg).

### Example: 6

9-Methoxymappicine ketone (**V**) (100 mg) in CHCl₃ (20 ml) was added to a solution of CAN (100 mg) in ethanol (10 ml). The mixture was stirred at room temperature for 2 hr. This was concentrated and to the concentrated mass water (20 ml) was added. This was extracted with EtOAc (3x10 ml), concentrated and purified by column chromatography over silica gel to afford 5-ethoxy-9-methoxymappicine ketone (92 mg).

### Example: 7

Camptothecin (**I**) (100 mg) was taken in AcOH (10 ml) and CAN (100 mg) was added. The mixture was stirred at room temperature for 8 hr. Water (20 ml) was added. The mixture was extracted with EtOAc (3x10 ml). The extract was concentrated and the impurities were removed by column chromatography over silica gel to yield 5-acetoxycamptothecin (93 mg).

### Example: 8

CAN (100 mg) was added to a mixture of camptothecin (100 mg) and propionic acid (10 ml) and this was stirred at room temperature for 8 hr. Water (20 ml) was added and the mixture was extracted with EtOAc (3x10 ml). The solvent was removed from the extract and the residue was subjected to column chromatography over silica gel to obtain 5-propionoxycamptothecin (90 mg).

### Example: 9

CAN (100 mg) was added to a well stirred solution of 9-methoxycamptothecin (100 mg) in acetic acid (10 ml) and the stirring was continued for 8 hr at room temperature. Water (20 ml) was added to the mixture and extracted with EtOAc (3x10 ml). The extract was concentrated and the impurities were removed by column chromatography over silica gel to yield 5-acetoxy-9-methoxycamptothecin (89 mg).

### Example: 10

To a solution of 20-O-acetyl camptothecin (100 mg) in propionic acid (10 ml) CAN (100 mg) was added and the mixture was stirred at room temperature for 8 hr. Water (20 ml) was added and the mixture was extracted with EtOAc (3x10 ml). The concentrated extract on chromatography over silica gel afforded 5-propionoxy-20-O-acetylcamptothecin (85 mg)

### Example: 11

To a solution of mappicine ketone (100 mg) in CHCl₃ (10 ml) benzoic acid (100 mg) and CAN (100 mg) were added. The mixture was stirrred for 6 hr at room temperature and water (20 ml) was added. This was extracted with EtOAc (3x10 ml) and the extract was concentrated. The residue was purified by column chromatography over silica gel to produce 5-benzoyloxymappicine ketone ( 99 mg).

### Example: 12

CAN (100 mg) was added to 9-methoxymappicine ketone (100 mg) dissolved in AcOH (10 ml). The mixture was stirred at room temperature for 6 hr. Water (20 ml) was added and the mixture was extracted with EtOAc (3x10 ml). The extract on purification by column chromatography over silica gel yielded 5-acetoxy-9-methoxymappicne ketone (89 mg).
**The main advantages of the present invention are:**
1. This is a direct method for preparation of 5-alkoxy and 5-acyloxy analogues of camptothecins and mappicine ketones.
2. The process is very simple.
3. The preparation of 5-alkoxy/acyloxy camptothecins and mappicine ketones is completed within a short period of time.
4. The reactions proceed at room temperature with an easily available and non-hazardous reagent (CAN).
5. The yields of the products are very high.

## Claims

1. A process for the direct preparation of 5-alkoxy and 5-acyloxy analogues of campthothecins or mappicene ketones comprising reacting campthothecin or mappicene ketone dissolved in an organic solvent or an acid with ceric ammonium nitrate in an organic solvent, concentrating the reaction mixture and adding water to the concentrate followed by extraction with ethyl acetate to obtain the desired product.

2. A process as claimed in claim 1 wherein the campthothecin is selected from the group consisting of campthothecin (I), 9-methyl campthothecin (II) and 20-O-acetyl campthothecin (III).

3. A process as claimed in claim 1 wherein the mappicene ketone used comprises 9-methoxymappicene ketone.

4. A process as claimed in claim 1 wherein the organic solvent used for the campthothecin solution is selected from the group consisting of dichloromethane, chloroform, tetrahydrofuran and any mixture thereof

5. A process as claimed in claim 1 wherein the organic acid used for the campthothecin solution is selected from acetic acid, propanoic acid and benzoic acid.

6. A process as claimed in claim 1 wherein the organic solvent used for the ceric ammonium nitrate solution is selected from the group consisting of methanol, propanol, isopropanol and ethanol.

7. A process as claimed in claim 1 wherein the reaction is carried out at a temperature in the range of 20 to 30°C for a period in the range of 2 to 10 hours

8. A process as claimed in claim 1 wherein the analogues obtained comprise the group of 5-methoxy campthothecin, 5-isopropoxy campthothecin, 5-ethoay-9 methoxy campthothecin, 5-methoxy mappicene ketone, 5-ethoxy-9-methoxy mappicene ketone, 5-acetoxy campthothecin, 5-acetoxy-9-methoxy campthothecin, 5-propionoxy-20-O-acetylcampthothecin, 5-benzoyloxymappicene ketone and 5 acetoxy-9-methoxymappicene ketone.

9. A process as claimed in claim 1 wherein the yield of the products comprises 72-86% for 5-alkoxy compounds and 71-82% for 5-acyloxy compounds.

10. A process as claimed in claim 1 wherein the solvent used for extraction is recovered.

## Patentansprüche

1. Verfahren zur direkten Herstellung von 5-Alkoxy- und 5-Acyloxyanalogen von Campthothecinen oder Mappicen-ketonen, umfassend das Umsetzen von Campthothecin oder von Mappicen-keton, gelöst in einem organischen Lösungsmittel oder einer Säure, mit Cerammonnitrat in einem organischen Lösungsmittel, Konzentrieren des Reaktionsgemisches und Zugeben von Wasser zu dem Konzentrat und anschließender Extraktion mit Ethylacetat zum Erhalt des gewünschten Produkts.

2. Verfahren nach Anspruch 1, wobei das Campthothecin aus der Gruppe ausgewählt ist, bestehend aus Campthothecin (I), 9-Methylcampthothecin (II) und 20-O-Acetyl-campthothecin (III).

3. Verfahren nach Anspruch 1, wobei das verwendete Mappicen-keton 9-Methoxymappicen-keton umfasst.

4. Verfahren nach Anspruch 9, wobei das für die Campthothecin-Lösung verwendete organische Lösungsmittel aus der Gruppe ausgewählt ist, bestehend aus Dichlormethan, Chloroform, Tetrahydrofuran und jegliches Gemisch davon.

5. Verfahren nach Anspruch 1, wobei das für die Campthothecin-Lösung verwendete organische Lösungsmittel aus Essigsäure, Propansäure und Benzoesäure ausgewählt ist.

6. Verfahren nach Anspruch 1, wobei das für die Cerammonnitrat-Lösung verwendete organische Lösungsmittel aus der Gruppe ausgewählt ist, bestehend aus Methanol, Propanol, Isopropanol und Ethanol.

7. Verfahren nach Anspruch 1, wobei die Reaktion bei einer Temperatur im Bereich von 20 bis 30 °C für eine Dauer im Bereich von 2 bis 10 h durchgeführt wird.

8. Verfahren nach Anspruch 1, wobei die erhaltenen Analogen die Gruppe von 5-Methoxycampthothecin, 5-Isopropoxycampthothecin, 5-Ethoxy-9-methoxycampthothecin, 5-Methoxymappicen-keton, 5-Ethoxy-9-methoxymappicen-keton, 5-Acetoxycampthothecin, 5-Acetoxy-9-methoxycampthothecin, 5-Propionoxy-20-O-acetylcampthothecin, 5-Benzoyloxymappicen-keton und 5-Acetoxy-9-methoxymappicen-keton umfasst.

9. Verfahren nach Anspruch 1, wobei die Ausbeute der Produkte 72-86 % für 5-Alkoxyverbindungen und 71-82 % für 5-Acyloxyverbindungen umfasst.

10. Verfahren nach Anspruch 1, wobei das zur Extraktion verwendete Lösungsmittel wiedergewonnen wird.

## Revendications

1. Procédé pour la préparation directe d'analogues 5-alcoxy et 5-acyloxy de camptothécines ou de mappicines cétones, comprenant la réaction de camptothécine ou de mappicine cétone dissoute dans un solvant organique ou un acide avec du nitrate d'ammonium cérique dans un solvant organique, la concentration du mélange réactionnel et l'addition d'eau au concentré suivie par l'extraction avec de l'acétate d'éthyle pour obtenir le produit souhaité.

2. Procédé selon la revendication 1, dans lequel la camptothécine est choisie dans le groupe constitué par la camptothécine (I), la 9-méthyl camptothécine (II) et la 20-O-acétyl camptothécine (III).

3. Procédé selon la revendication 1, dans lequel la mappicine cétone utilisée comprend de la 9-méthoxymappicine cétone.

4. Procédé selon la revendication 1, dans lequel le solvant organique utilisé pour la solution de camptothécine est choisi dans le groupe constitué par le dichlorométhane, le chloroforme, le tétrahydrofuranne et tout mélange de ceux-ci.

5. Procédé selon la revendication 1, dans lequel l'acide organique utilisé pour la solution de camptothécine est choisi parmi l'acide acétique, l'acide propanoïque et l'acide benzoïque.

6. Procédé selon la revendication 1, dans lequel le solvant organique utilisé pour la solution de nitrate d'ammonium cérique est choisi dans le groupe constitué par le méthanol, le propanol, l'isopropanol et l'éthanol.

7. Procédé selon la revendication 1, dans lequel la réaction est effectuée à une température dans la gamme de 20 à 30 °C pendant une période dans la gamme de 2 à 10 heures.

8. Procédé selon la revendication 1, dans lequel les analogues obtenus comprennent le groupe de 5-méthoxy camptothécine, 5-isopropoxy camptothécine, 5-éthoxy-9-méthoxy camptothécine, 5-méthoxy mappicine cétone, 5-éthoxy-9-méthoxy mappicine cétone, 5-acétoxy camptothécine, 5-acétoxy-9-méthoxy camptothécine, 5-propionoxy-20-O-acétylcamptothécine, 5-benzoyloxymappicine cétone et 5-acétoxy-9-méthoxymappicine cétone.

9. Procédé selon la revendication 1, dans lequel le rendement en les produits comprend 72 à 86 % pour les composés 5-alcoxy et 71 à 82 % pour les composés 5-acyloxy.

10. Procédé selon la revendication 1, dans lequel le solvant utilisé pour l'extraction est récupéré.
